# EUROPEAN PATENT SPECIFICATION

(11) **EP 2 399 626 B1**
(45) Date of publication and mention of the grant of the patent: **19.06.2013**
(21) Application number: 10167585.8
(22) Date of filing: 28.06.2010
(51) Int. Cl.: A61M 5/168, A61M 5/142, A61M 5/14, A61M 5/36

(54) **Sensor for use in liquid medicament delivery systems**
Sensor zur Verwendung in einem System zur Verabreichung von flüssigen Medikamenten
Capteur à utiliser dans des systèmes d'administration de médicament liquide

(43) Date of publication of application: 28.12.2011
(73) Proprietor: F. Hoffmann-La Roche AG, 4070 Basel (CH); Roche Diagnostics GmbH, 68305 Mannheim (DE)
(72) Inventor: Leuenberger, James, 3412, Heimiswil (CH)
(74) Representative: Rentsch Partner AG

(56) References cited:
- EP-A1- 1 818 664
- EP-A1- 2 022 519
- US-A- 4 238 207
- US-A- 4 554 837
- US-A1- 2008 294 094

## Description

### Field of the Invention

The invention relates to sensor devices for use in liquid medicament delivery systems, with a micro-fluidic chamber and optical detection system, to infusion pump devices and liquid medicament delivery systems with such sensor devices, and the use of such sensor devices for measuring the pressure and/or the presence of air bubbles in a fluidic system.

### State of the art

Devices for the automated release of liquid medicaments are generally used with patients who have a continuous, and in the course of the day varying, need of a medicine that can be administered by subcutaneous infusion. Specific applications are for example certain pain therapies and the treatment of diabetes. In such cases, computer controlled infusion pump devices are used, which can be carried by the patient on the body, and which contain a certain amount of liquid medicament in a medicine reservoir. The medicine reservoir often comprises medicine sufficient for one or several days. The liquid medicament is supplied to the patient's body from the medicine reservoir through an infusion cannula or an injection needle.

Particularly in self-administration of medicaments, for example insulin, the patients using the medicament in question and administering it themselves by means of an infusion pump device tend to emphasize convenience and discretion. As a consequence, the acceptable dimensions of such infusion pump devices are limited in order not be evident through clothing and to be carried as comfortably as possible. In an advantageous type of infusion pump device, the liquid medicament is obtained by a downstream pump from a flexible container. Flexible containers have the advantage of a smaller volume surplus of the container in relation to its content, which reduces the manufacturing costs and enables design of infusion pump devices of smaller overall dimensions.

In the context of liquid medicament administration via an infusion pump device, sensor devices can be used for controlling the dosing, monitoring the correct operation of the system, and for fast detection of faults and hazards, such as occluded infusion lines or cannulae, empty containers, or malfunctioning pump systems. A pressure sensor device is typically arranged in the fluid path downstream of a pump device and upstream of an infusion cannula.

Such pressure sensor devices typically comprise a micro-fluidic chamber filled with liquid and fluidly connected to the fluidic system. The chamber is covered by a flexible, resilient membrane, such that a pressure difference between the fluidic pressure inside the sensor chamber and the outside (such as atmospheric) pressure will temporarily deform the membrane. The resulting deflection of the membrane can then be measured by suitable means in order to determine the internal pressure of the fluidic system.

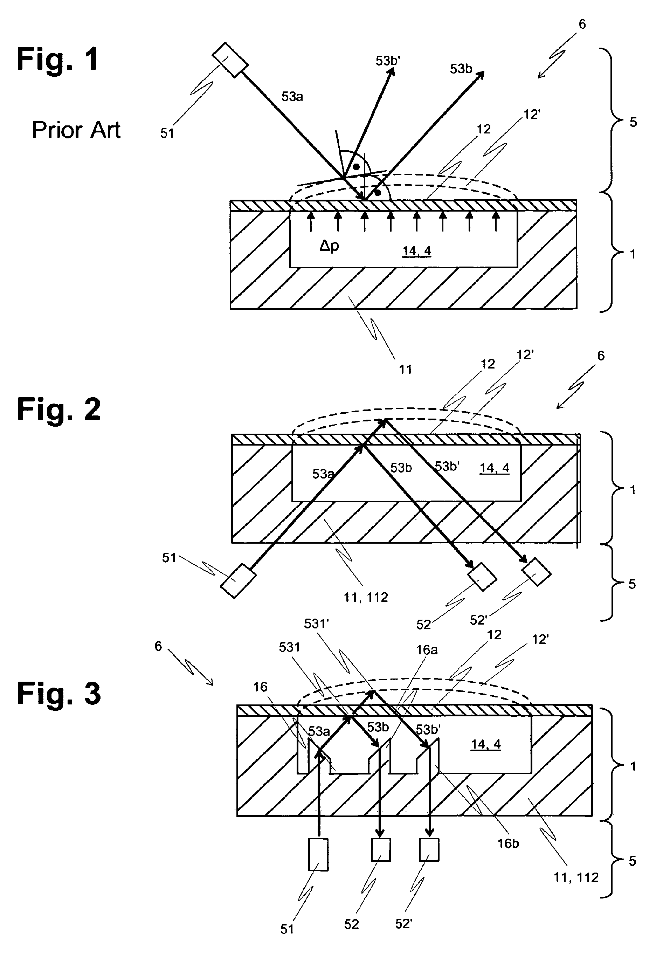

A suitable approach to measure the deformation of the membrane is optical detection of a light beam reflected off of the membrane. Such pressure sensor devices are, for example, disclosed in "A microfluidic experimental platform with internal pressure measurements," M. J. Kohl et al., Sensors and Actuators A 118 (2005), pp. 212-221. Figure 1 schematically shows such a pressure sensor device 6 according to the prior art. A micro-fluidic chamber 1 that is connected to a fluidic system comprises a rigid bottom substrate 11 and a flexible, resilient top cover 12, for example, a membrane. An optical detection system 5 is arranged to measure a deformation of the cover membrane 12 by determining the interaction of a light beam 53a with the cover membrane 12. For that purpose a light emitting device 51, e.g. a laser diode, directs a light beam 53a at a certain angle onto the surface of the cover membrane 12, where it is reflected 53b. The pressure difference Δp between the inner volume 14 of the micro-fluidic chamber and the outer environment acts on the cover membrane 12, and deforms it to a certain extent 12', depending on the pressure difference. As a result the angle of the reflected light beam changes and the beam is transversely shifted. By observing the position of the reflected light beam 53b, 53b', the deformation of the cover membrane can be measured, and based on the obtained results a pressure difference value can be determined.

US 4554837 discloses a reflective optical pressure sensor for measuring the pressure of a liquid. The sensor comprises a light source and a capillary formed in a transparent solid having a reflective surface positioned in the path of light from the light source. A light detector is positioned in the path of light originating from the light source which has been reflected by the reflective surface of the capillary. The inlet end of the capillary is open for communication with liquid to be monitored for pressure, and the other end of the capillary is closed or communicates with a closed reservoir. If the pressure of the liquid to be monitored increases, increasing amounts of the liquid pass into the capillary passageway, blocking or reducing light flow to the light detector.

US 2008/0294094 A1 discloses a pressure sensor for an infusion pump device. A capillary tube fluidly connects a fluid channel through which the liquid medicament is delivered to a patient with a flexible membrane that sealingly closes an air cavity. A pocket of air is trapped in the capillary tube to separate the liquid medicament in the fluid channel from the flexible membrane. When the air pressure in the capillary tube rises in response to a rise in the fluid pressure in the flow channel, the flexible membrane flexes into the air cavity, and finally touches the wall surface of the air cavity. A light beam is reflected on the wall surface of the air cavity and is detected, in order to observe the deformation of the flexible membrane.

US 4238207 discloses a fluid filter device for filtering parenteral solutions, which separates gas from liquid and vents the separated gas from the filter. A membrane that is permeable only for liquid is arranged in a closed filter housing, thereby dividing the housing into a first chamber with an inlet, and a second chamber with an outlet. Another membrane, which is permeable only for gases, is arranged between the first chamber and a one-way venting valve. The liquid enters the first chamber through the inlet, passes through the liquid-permeable membrane into the second chamber, driven by the pressure differential across the membrane, and leaves the filter device through the outlet. Gas bubbles are retained in the first chamber, and leave the chamber via the gas-permeable membrane and the venting valve toward the environment. To support the fragile liquid-permeable membrane against the pressure differential across the membrane, spaced parallel ribs are provided in the second chamber that closely underlie and support the filter membrane.

For being able to observe the reflected light beam, a detector in an optical detection system 5 must be designed to be movable, or a multiplicity of detectors at different positions and at different angles must be included in a device according to the prior art. Both of these aspects make such sensor devices expensive and difficult to make.

The flexible, resilient top cover membrane 12 is rather delicate and thus prone to damage. A compromised or even damaged cover membrane would lead to erroneous pressure measurements and/or to leaking of the fluidic system, both of which are not acceptable. Consequently the top cover membrane 12 should be protected from mechanical damage as well as other detrimental environmental influences. At the same time the flexible top cover 12 has to remain accessible to the optical detection system 5.

The optical detection system 5 can be arranged within a suitable protective cover for the membrane 12. However, since a fluidic system of a liquid infusion pump system including any pressure sensor device is generally designed as a disposable part, for hygienic reasons, such a solution is very expensive, since any light emitting and receiving devices of the detector system 5 would have to be discarded together with the micro-fluidic chamber.

It is important that the micro-fluidic chamber of the pressure sensor device is free of air bubbles, in order to avoid systematic or random measurement errors. Air bubbles in the micro-fluidic sensor chamber (and more generally, anywhere within a fluidic system) reduce the stiffness of the fluidic system, and thus delay the response of the sensor to pressure changes that may occur if the fluidic system becomes occluded. The resulting irreproducible measurement errors can reduce the dosing accuracy of an infusion pump device, and increase the response time to an occlusion event.

Air bubbles present in a fluidic system of an infusion pump devices, particularly in the pump system, but also in other components such as the container, also cause further problems. If air bubbles remains in the fluidic system, they may be administered instead of the liquid medicament, which leads to potentially dangerous dosing errors. Furthermore, the administration of air into a patient's body should be generally avoided for medical reasons.

A further problem of fluidic systems, particularly in infusion pump devices, is the dead volume in the fluidic system. Said dead volume cannot be used, meaning that it cannot be emptied or drained completely. Thus, the dead volume considerably increases the effective cost per dose and thus of the overall therapy cost, since a certain percentage of the liquid medicament inevitably remains in the fluid system and has to be disposed. This negative cost effect is particularly important for expensive medicaments. Furthermore, the relative portion of a certain dead volume of a given overall reservoir size increases with decreasing absolute reservoir size. Minimizing the dead volume therefore becomes more and more important with decreasing reservoir size.

To avoid air bubbles in the micro-fluidic chamber when the fluidic system is filled the first time, the so-called priming of the system, the chamber has to be filled in a controlled manner. However, this goal may be impeded by an uncontrolled orientation of the micro-fluidic chamber in space during this first filling procedure, since the gravitation field leads to buoyancy forces that act on the air bubbles. Depending on the orientation and the design of the micro-fluidic chamber, air bubbles may be caught in certain areas of the chamber.

Due to the many problems that may be caused by air bubbles in a fluidic system, as explained above, there is an urgent need for reliable and inexpensive sensors that are able to detect air bubbles in fluidic systems.

For the present specification the meaning of the term "air" shall not only include air as such, but any gas or composition of gases that may be present in a fluidic system, particularly pure nitrogen or other protective gases.

### Objects of the Invention

It is an object of this invention to provide an advantageous sensor device for use in a fluidic system, particularly in an infusion pump device for liquid medicaments, that overcomes one or more of the above-mentioned and other problems.

It is a further object of this invention to provide an advantageous sensor device for use in a fluidic system that can be used as a pressure sensor and/or an air bubble sensor.

It is yet another subject of the invention to provide a sensor device with a less complex optical detection system. Advantageously in such a sensor device the optical detection system can be easily aligned to the other components.

It is also an object of the present invention to provide a sensor device that insensitive to small variations in the assembly of its components.

In a sensor device according to the invention any sensitive parts should be protectable against mechanical damage. Any parts of the sensor that come into contact with liquid are advantageously arranged in a separate subunit that can be releasably attached to a reusable subunit.

Such a sensor device should provide a small dead volume, and it should be fillable without air remaining in the chamber essentially independent of its orientation in space. It should be producible at low cost in a large-scale manufacture.

In addition, it is an object of the present invention to provide an infusion pump device or parts of an infusion pump device, and liquid medicament delivery systems with a sensor device according to the invention.

These and other objects are achieved by a sensor device, an infusion pump device, and a liquid medicament delivery system according to the independent claims. Advantageous embodiments are given in the dependent claims.

### Summary of the invention

A sensor device according to the invention for use in a liquid medicament delivery system has a micro-fluidic chamber comprising a rigid bottom substrate and a cover, and an optical detection system that is arranged to emit one or more light beams toward the cover and to observe the one or more light beams reflected from the cover. The optical detection system is arranged on a side of the bottom substrate opposite to the cover.

Advantageously, the one or more light beams impinging on the cover and/or the one or more reflected light beams pass through the bottom structure of the micro-fluidic chamber. A reusable part comprising the optical detection system can be coupled to a disposable part comprising the micro-fluidic chamber.

The cover is a flexible, resilient cover membrane, and the optical detection system is arranged to determine a deformation of the cover membrane. Such an advantageous sensor device may be used as a pressure sensor device.

When there is no pressure difference between the external pressure and the internal pressure of the fluidic system, the top cover membrane remains flat. In the case of a positive pressure difference, the membrane will bulge outwards. The resulting displacement of the outer surface of the flexible membrane then is used to determine the current pressure difference.

In such a pressure sensor device according to the invention the optical detection system measures the deformation of the cover membrane through the bottom structure and the liquid-filled inner volume of the chamber. Thus, the outer side of the sensitive membrane can be protected against mechanical damage and environmental influences by placing an inexpensive protective cover over the membrane.

In another advantageous embodiment of a sensor device, the wavelength of the emitted light beams is chosen such that a liquid in the micro-fluidic chamber shows a high absorption coefficient at this wavelength. Such an embodiment is particularly advantageous as an air bubble sensor device.

In yet another advantageous embodiment of a sensor device according to the invention, the optical detection system comprises two or more photo sensors or a photo sensor array.

Advantageously, a sensor device according to the invention is adapted such that the one or more light beams are reflected on a fluid-contacting surface of the cover.

The liquid in the fluid system is generally water or an aqueous solution. For water, advantageous wavelengths are for example 630 nm or 1400 nm. An absorption maximum for a typical insulin formulation is given at 270 nm. Thus such a wavelength is particularly advantageous for use with insulin pump systems.

When an air bubble passes a light beam, the absorbance along the light path is considerably reduced, and more light is detected. Thus such a sensor device can be used to identify the presence of air in the path of a light beam, and thus is able to detect the passage of an air bubble.

If the device according to the invention comprises two or more photo sensors, or even a photo sensor array, the measured signal on the different detectors is assessed in relation to the signal on the other detectors, in order to measure of the deflection of a cover membrane, and thus to determine the pressure in the fluidic system. For detecting the presence of air in the light path on the other hand, and thus the detection of an air bubble, the absolute intensity of the detected light is assessed. These two channels of information are essentially independent from each other. In case there is only one photo sensor used in a sensor device according to the invention, it is also possible to detect an air bubble. An air bubble will result in an increase of the detected light signal, while a deflection of the cover membrane will deviate the light beam from the light sensor, thereby decreasing the detected light signal. Consequently, a sensor device according to the invention has the particular advantage that it can be realized in such a way that it can be used as a pressure sensor and an air bubble sensor at the same time.

In an advantageous embodiment of such a sensor device according to the invention, the bottom structure comprises prism-like structures for coupling a light beam from a bottom structure material into a liquid in an inner volume of the micro-fluidic chamber, and/or from the liquid into the bottom structure material.

One of the principle advantages of using such prismatic structures is that the path of the returning reflected light beam is defined by the prism structures. Optical sensors can be arranged under the prism structures. Thus, the optical detection system can be realized as a static system without moving parts. Only a small, defined number of detectors is needed, which can be easily aligned in regard to the prism structures.

The correct function of the optical detection system in a sensor device according to the invention is insensitive to small variations in the position and orientation of the detectors. Thus the necessary accuracy in the manufacturing and assembling process is decreased, which reduces the overall cost of the sensor device.

In one advantageous embodiment, the top cover membrane of the sensor device is gas-permeable, which has the advantage that any air bubble remaining in the sensor device can drain through the cover membrane.

In a particularly advantageous embodiment of the sensor device according to the invention, one or more walls or fillings are positioned in the chamber, the walls or fillings defining a fluid channel therebetween such that the fluid channel extends from an inlet of the chamber to an outlet of the chamber. Each of the walls or fillings has a height less than a height of the chamber defined by the distance between the bottom substrate and the cover membrane, so as to define a fluid gap between a top surface of each wall or filling and the cover membrane. The dimensions of the walls or fillings and the chamber are chosen such that the fluid gap will be filled with liquid by capillary forces via the fluid channel when liquid is introduced into the fluid chamber. In other words, the fluid gap adjacent to a section of the fluid channel filled by a liquid introduced into the fluid chamber will be filled with said liquid by capillary forces.

Such a sensor device according to the invention has a considerably reduced dead volume compared to the state of the art. In addition, in the case of a negative pressure difference, where the cover membrane will be displaced inwards toward the chamber, the walls can in certain embodiments support the cover membrane, thereby avoiding an occlusion of the micro-fluidic chamber by the membrane itself.

In an even more advantageous variant of such a sensor device according to the invention, prism-like structures are provided in the walls or fillings for coupling a light beam from a bottom structure material of the wall into a liquid in an inner volume of the micro-fluidic chamber and/or from the liquid into the bottom structure material.

Advantageously, at least a part of the surface of the bottom structure, and/or the walls, and/or the top cover membrane facing toward an inner volume of the chamber is hydrophilic. This increases the capillary forces in the gap, particularly for aqueous liquids.

The height of the gap advantageously lies between about 0.02 and about 0.2 mm, and more advantageously between 0.05 and 0.15 mm. The fluid channel can have a curved or meander-like shape, or may be straight. Some embodiments of sensor devices according to the invention have two or more fluid channels.

An additional conduit bypassing the micro-fluidic chamber of a sensor device according to the invention can be provided, by fluidly connecting an inlet conduit and an outlet conduit of the chamber, with the advantage of increasing the flow capacity of the micro-fluidic chamber and thus of the sensor device. Advantageously the width of an inlet of the bypass conduit is smaller than the width of the inlet conduit, to prevent air bubbles from entering the bypass conduit.

In another advantageous variant of a sensor device according to the invention, one or more additional outlets conduits branch off from the fluid channel.

Since the micro-fluidic chambers of the sensor devices according to the invention can be manufactured in large numbers and on continuous production lines, the effective cost per piece are sufficiently low that they can be realized as single-use products.

An infusion pump device according to the present invention and a liquid medicament delivery system according to the present invention comprise a sensor device according to the invention as discussed above.

Another advantageous aspect of the invention is the use of a sensor device according to the invention as discussed above for measuring the pressure in a fluidic system, and/or for measuring the presence of air bubbles in a fluidic system.

### Brief description of the drawings

In order to facilitate a fuller understanding of the present invention, reference is now made to the appended drawings. These references should not be construed as limiting the present invention, but are intended to be exemplary only.
- Figure 1: schematically shows a sensor device as known from the prior art in a cross-sectional view, with a micro-fluidic chamber and an optical detection system.
- Figure 2: schematically shows an embodiment of a sensor device according to the invention, in a cross-sectional view.
- Figure 3: schematically shows another embodiment of a sensor device according to the invention, in a cross-sectional view.
- Figure 4: schematically shows a typical embodiment of a micro-fluidic chamber for use in a sensor device according to the invention, (a) in a top view, and (b) in a cross-section along plane A-A. Figure 4(c) shows a detail view of Figure 4(b).
- Figure 5: shows the distribution of liquid in an embodiment of a micro-fluidic chamber for use in a sensor device according to the invention, during the filling of the chamber in a real experiment, in two subsequent stages.
- Figure 6: schematically shows an advantageous embodiment of a sensor device according to the invention, with prism-like structures in combination with a meandering fluid path, (a) in a top view, and (b) in a cross-section along line A-A.
- Figure 7: shows different alternative variants of prism-structures for use in a sensor device according to the invention as shown in Figure 6.
- Figure 8: depicts four other possible exemplary embodiments of micro-fluidic chambers for use in a sensor device according to the invention.
- Figure 9: schematically shows the sensor device according to Figure 6, with an air bubble present in the fluid channel.
- Figure 10: shows (a) an embodiment of a micro-fluidic chamber for use in a sensor device according to the invention with additional outlets along the fluidic channel in the chamber, providing bubble-trap capabilities, and (b) an embodiment of a micro-fluidic chamber for use in a sensor device according to the invention with a bypassing additional conduit.

### Description of embodiments of the invention

A first embodiment of a sensor device 6 according to the invention is depicted in Figure 2, in a cross-section through the micro-fluidic chamber 1 of the sensor device 6. The micro-fluidic chamber 1 comprises a bottom structure 11 and top cover membrane 12, which define an inner volume 14 of the chamber 1. The chamber is fluidly connected to a fluidic system (not shown). The cover membrane 12 is flexible and resilient.

During operation of the sensor device according to the invention as a pressure sensor, the inner volume 14 of the micro-fluidic chamber 1 is filled with liquid. The pressure difference Δp between the liquid of the inner volume 14 and the pressure (such as atmospheric pressure) of the outer environment acts on the cover membrane 12, and bulges the membrane outwards 12'. When the pressure difference drops, the deformed and thus biased membrane returns toward the unloaded, flat state. The amount of deformation of the cover membrane 12 depends on the pressure difference Δp. Thus the deformation of the membrane 12 can be used to measure the pressure difference.

Using an elastomeric polymer as the material for the cover membrane, a circular membrane with a diameter in a range of, for example, 2 to 7 mm can be used to measure pressure differences between 0 and 200 mbar. For larger pressure differences, smaller diameters are used, since the deformation of the membrane should not exceed certain mechanical limits defined by the materials used.

In the shown embodiment of a sensor device according to the invention, a light emitting device 51, for example a laser diode or a light emitting diode emits a collimated light beam 53a toward the top cover membrane 12. The path of the light beam 53a runs through the transparent bottom structure 11 of the chamber and the liquid medium 4 in the inner volume. The bottom structure is advantageously manufactured from a transparent polymer material, such as for example PMMA or polystyrene.

Upon transition of the light from the bottom structure material 112 to the liquid medium 4 the light beam is refracted, depending on the ratio of refraction indices of the two materials. The light beam 53a is then reflected by the inner and/or the outer surface of the cover membrane 12. The cover membrane may be metal vapor coated to increase reflection. The reflected beam 53b passes again the liquid 4 and the bottom structure material 112, and finally is received by a photo sensor 52, 52', for example a for example a photo diode or a photo transistor.

Both the light emitting device 51 and the photo sensors 52, 52' are located on the side of the micro-fluidic chamber opposite to the membrane 12. Therefore, a pressure sensor device 6 according to the invention as shown in Figure 2 has the particular advantage over the prior art that the optical detection system 5 does not need to access to the outer surface of the membrane 12. Thus, by coupling the light through the bottom structure 11 of the chamber, the membrane 12 can be provided with a protective cover or casing (not shown), shielding the membrane 12 from mechanical damage and environmental influences. There is no need for high accuracy of the positioning or the optical characteristics of the protective cover, which reduces the manufacturing costs.

Furthermore the protective cover can be used to restrict the deformation of the membrane to a certain maximum level, thereby preventing the membrane from irreversible damage due to overpressure in the fluidic system. Such an advantageous, additional use of the cover would not be possible if the membrane would have to be optically accessible from the outside.

The optical detection system can be part of a reusable subunit of the sensor device according to the invention, which can be releasably attached to the lower side of the micro-fluidic chamber, opposite to the cover membrane, which is preferably permanently and inaccessibly protected by a cover. The chamber can be part of a disposable subunit of the sensor, comprising all parts that come into contact with the liquid medicament and must not be reused. In a particularly advantageous embodiment, the optical detection system is part of a reusable unit of an infusion pump device, which is releasably coupled to a disposable unit of the pump device, including the fluidic system with the micro-fluidic chamber.

When the cover membrane is deformed 12', the path of the reflected light beam is shifted and deflected 53b', depending on the degree of deformation. A photo sensor 52 can only receive the light beam 53b as long as it is in a certain spatial range. Thus in a setup of a sensor device with one single photo sensor, is only possible to determine if the deformation of the cover membrane 12, or the corresponding pressure differential, lies in a predefined target range, corresponding to the spatial detection range of the photo sensor. In such an embodiment the optical detection system 5 thus delivers a binary on/off signal correlated to a certain pressure threshold, which can be used by a control unit of an infusion pump system. Such a relatively simple system is completely sufficient to detect an occlusion in a fluid line.

In the embodiment shown in Figure 2, two photo sensors 52, 52' are provided. A first sensor 52 receives the reflected light beam 53b over a first range of membrane deformations, including the state with undeformed membrane 12, equivalent to zero pressure difference. A second photo sensor 52' receives the reflected light beam 53b' over an adjacent range of deformation, including a maximum allowable deformation state of the membrane 12'.

Instead of one or two photo sensors, a multitude of sensors may be used. In a particularly advantageous embodiment a CCD sensor array 52 is used to detect the reflected light beam, which will allow a determination of the membrane deformation in high transversal resolution, and thus will allow the measurement of the pressure difference with increased precision. Such an embodiment delivers more detailed information on the pressure and thus is advantageous in case a control unit uses the pressure values to calculate and/or monitor the current flow of liquid and the administered dose of liquid medicament.

The use of a CCD sensor array has the further advantage that deviations within a certain range of the angular orientation of the array in regard to the micro-fluidic chamber can be taken into account by a calibration measurement. This increases the precision of the measurements, and allows to correct variations of the alignment of the reusable optical detection system when coupled to a new sensor chamber.

The point of reflection 531 of the light beam 53a may be chosen on any position of the cover membrane 12. The closer said position 531 to the center of the membrane, the larger the deflection of the membrane 12 in the vertical direction (perpendicular to the undeflected membrane), which corresponds to larger transversal shift of the reflection point 531' and thus of the reflected light beam 53b'. The closer the position 531 to the border of the chamber, the larger the change of inclination of the membrane surface 12 and thus of the reflection angle upon deformation, which also corresponds to a larger shift of the reflected light beam 53b. To obtain maximum resolution, an optimum has to be chosen from the position and orientation of the light emitting device 51 and photo sensors 52, 52'. A good position is for example a reflection point at 50% of the radius of the micro-fluidic chamber.

Another advantageous embodiment of a sensor device according to the invention is shown in Figure 3. In this particular embodiment, the bottom structure 11 is provided with prism-like structures 16, 16a, 16b protruding from the bottom structure 11. A first prism-like structure 16 is used to couple a collimated light beam 53a emitted by light emitting device 51 from the medium 112 of the bottom structure into the liquid medium 4 in the chamber, allowing for steeper incident angles of the light beam 53a on the cover membrane 12.

Two other prism-like structures 16a, 16b are used to couple the reflected light beam 53b, 53b' back into the bottom structure material 112. Instead of two prism-like structures and two photo sensors 52, 52', as shown in Figure 3, a multitude of such prisms and/or a multitude of photo sensors including a CCD sensor array can be applied to increase resolution.

An important advantage of such an embodiment of a sensor device according to the invention is the insensitivity of the optical detection system 5 to small deviations of its orientation in regard to the micro-fluidic chamber. Such small variations may result when a reusable part and a disposable part are coupled, even when guiding structures are provided.

Since the reflected light beams 53b, 53b' are guided by the prism structures 16a, 16b to the corresponding optical sensors 52, 52' placed beneath the prism structures, small lateral displacements of the sensors will not affect the accuracy of the optical detection system. Even larger displacements, affecting the alignment of the prism and the sensor and thus the amount of light detected by the sensor, can be corrected, since all sensors will be subject to the same amount of misalignment.

Since all detectors are oriented in the same direction, the assembly of the device can be carried out by standard automatic placement machine, which considerably reduces manufacturing costs.

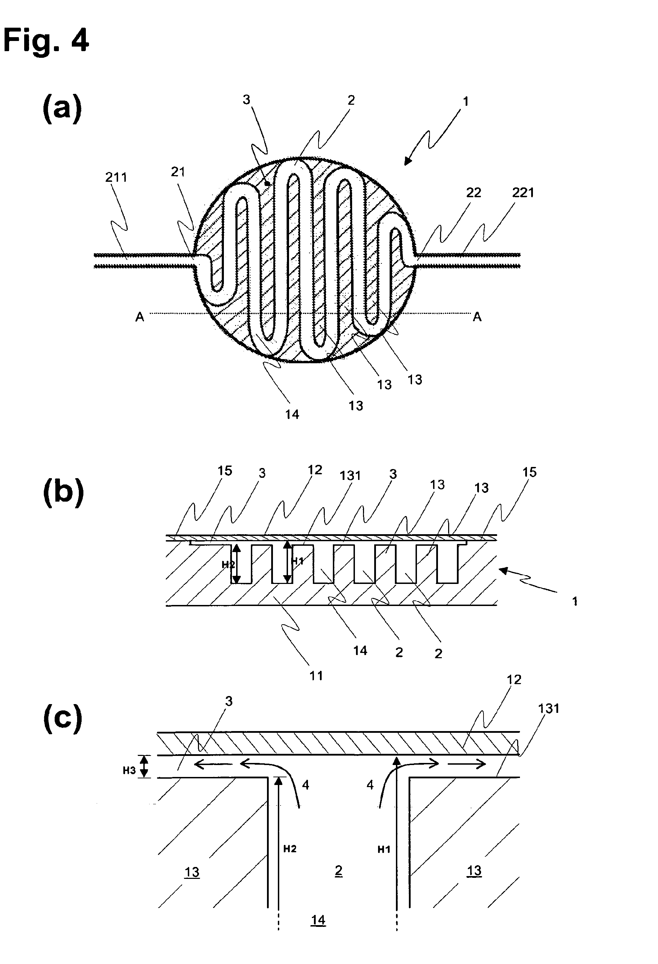

A particularly advantageous embodiment of a micro-fluidic chamber that is suitable for use in a sensor device according to the invention is shown in Figure 4. The circularly shaped fluid chamber 1 comprises a bottom substrate 11 and a top cover 12. The top cover 12 is spaced from the bottom substrate 11 by a certain height H1, thus defining an inner volume 14 of the chamber 1. Eight walls 13 are arranged in the fluid chamber 1, and define a meander-like fluid channel 12 that runs from an inlet 21 to an outlet 22 located on the opposite side of the chamber 1. Thus the inlet conduit 211 and the outlet conduit 221 are fluidly connected by the fluid channel 12.

The height H2 of the walls 13 is less that the overall height H1 of the chamber 1. As a result there is a fluid gap 3 between the top cover 12 and the upper surface 131 of the walls 13, with a height H3 = H1 - H2. The dimensions of the chamber and the walls, particularly the heights H1, H2, H3 are chosen such that there are non-negligible capillary forces acting on a fluid 4 present in the micro-fluidic chamber 1. Fluid 4 in the fluid channel 2 will be dragged by said capillary forces into the fluid gap 3.

The specific dimensions depend on one hand on the liquid used, and on the other hand on the properties of the surfaces of the top cover 12 and the top 131 of the walls 13, since this will eventually define the interface tensions between liquid, surfaces, and gas/air in the chamber, which then will define the effective capillary forces for a certain geometric setting of a micro-fluidic chamber. Since in most cases liquid medicaments are aqueous solutions, it is preferable that at least the most relevant surfaces, namely the surface of the top surface 131 of the wall 13 and the surface of the top cover 12 facing toward surface 131, are hydrophilic with a contact angle < 90°, in order to increase the overall capillary effect. For aqueous solutions a preferred range for the height H3 of the gap 3 lies between 20 and 200 µm, and preferably between 50 and 150 µm.

The dimensions of the chamber 1 and the fluid channel 2 are less critical. A typical diameter of a micro-fluidic chamber 1 may for example lie between about 2 to 10 mm. The fluid channels may have a width of for example 0.1 to 1 mm, while the height H2 of the walls 13 lies in a range between 0.25 to 5 mm, and preferred lies between 0.5 and 1 mm. The aspect ratio between the width of the fluid channel 2 and the height H2 can lie between 0.25 and 5, and is preferably about 1.

When a micro-fluidic chamber 1 is filled through inlet 21 with a liquid 4, the liquid will flow essentially along the fluid channel 2. The capillary forces will drag liquid 4 in the fluid channel 2 into the adjacent sections of the gap 3, effectively supplanting air present in the gap. It is energetically much more favorable for air to form spherical bubbles with minimum surface toward the hydrophilic surroundings, and thus no air bubbles remaining in the gap 3.

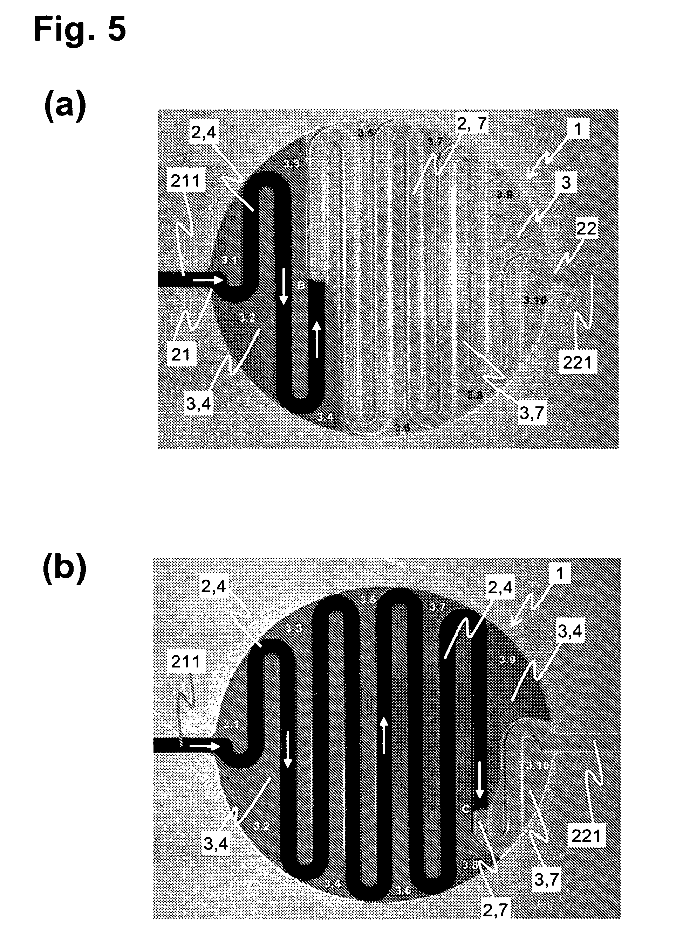

The first filling of such a micro-fluidic chamber 1 suitable for use with a sensor device according to the invention is experimentally demonstrated in Figure 5. In Figure 5(a) an aqueous liquid 4 has flown downstream through inlet conduit 211 and inlet 21 into the fluid channel 2, and is currently at a position B. Due to the capillary forces in the gap 3, the liquid 4 flows into the sections 3.1, 3.2 3.3, 3.4 of gap 3 adjacent to the fluid channel 2 already filled. In the gap the surrounding sections of the fluid channel 2 downstream of position B that are still filled with air or gas 7 limit the further flow of the liquid. Thus the gap 3 is filled section by section. Figure 5(b) shows a later stage, where the liquid 4 has proceeded in the fluid channel 2 to a position C. All sections of the gap 3 are filled with liquid 4, except section 3.10, which has not yet come into contact with the liquid and is still filled with air 7. It is clearly visible in Figure 5 that no air 7 remains in the part of the chamber that has already been filled by liquid 4. When finally the liquid will have reached the outlet 22 and the outlet conduit 221, the micro-fluidic chamber 1 will be filled completely. No air 7 remains in the micro-fluidic chamber.

Air bubbles in the gap are energetically less preferable than air bubbles in the fluid channel 2. As a consequence also no air bubbles will form in the gap 3 at a later stage, and if they do they will migrate into the fluid channel 2. Air bubbles in the fluid channel 2, on the other hand, will not enter the gap 3 for energy reasons, but will be transported away by the liquid stream.

The shown capabilities of a micro-fluidic chamber 1 are independent from its orientation in space. Since the capillary forces and interface tensions responsible for the smooth filling of the gap are much stronger than the gravitational force acting on the liquid, and the buoyancy force acting on the air bubbles in the liquid, the micro-fluidic chamber finally will be completely filled with liquid 4 independent on its orientation. Thus the filling behavior of such a micro-fluidic chamber are predictable and reproducible, which is very advantageous for use in a sensor device according to the invention.

Since the operational internal volume of a micro-fluidic chamber is smaller than that of a hollow micro-fluidic chamber with similar dimensions, the dead volume - the portion of the fluid volume in a fluid system that can never be drained and eventually will be lost when the sensor device is discarded - is considerably reduced.

A further advantage of the disclosed micro-fluidic chamber is the fact that an air bubble that enters the chamber through the inlet will be guided through the fluid channel to the outlet. Since the effective cross-sectional area of the fluid channel is essentially constant over its length, the liquid flow is also constant over its length, and does not drop at certain positions. Thus, bubbles cannot be caught in the fluid chamber.

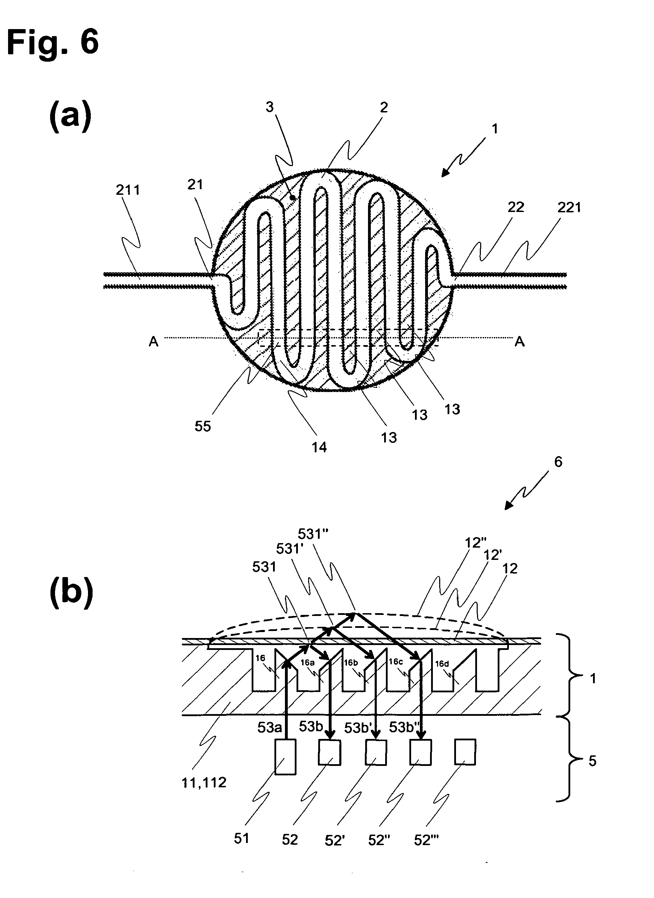

A particularly advantageous sensor device 6 is disclosed in Figure 6 and exemplifies a particular useful embodiment of a sensor device, where the advantageous optical detection scheme of a sensor device according to the invention is combined with the advantages of a micro-fluidic chamber with meandering fluid channel 2. The micro-fluidic chamber 1 of such a sensor device essentially corresponds to the embodiment shown in Figure 4, while detection is realized similar to Figure 3.

In a rectangular detection area 55 the walls 13 of the chamber 1 are reduced to prism-like structures 16, 16a, 16b, in order to couple an incident light beam 53a propagating in the wall into the liquid 4, and to couple the reflected light beam 53b, 53b' back into the walls and the bottom structure 11. The prism structures form only a narrow gap in the walls 13. This prevents that the fluidic resistance along the detection area falls below the flow resistance along the fluidic channel 2.

A light-emitting device 51 is arranged directly below the first prism-like structure 16, and emits a collimated light beam 53a that propagates in the wall until it reaches the inclined prism surface, where it is refracted toward the reflection point 531. After reflection on the cover membrane 12, the reflected light beam 53b impinges on the prism surface of a second prism-like structure 16a, where it is refracted toward a photo sensor 52 arranged directly below the prism 16a.

When the pressure difference increases, the membrane is deformed, and the reflection point 531' moves to the right. The reflected light beam 53b now impinges on the prism surface of the third prism-like structure 16b, and reaches a second photo sensor 52'.

With further increasing pressure difference the reflected light beam reaches next prism 16c, etc.

The inclination angle and the general dimensions of the prismatic structures depend on the dimensions and properties of the micro-fluidic chamber, on the wave-length used for the light beam, the refraction indices of the bottom structure material and the liquid medium, and other factors, and have to be adopted for a particular sensor geometry.

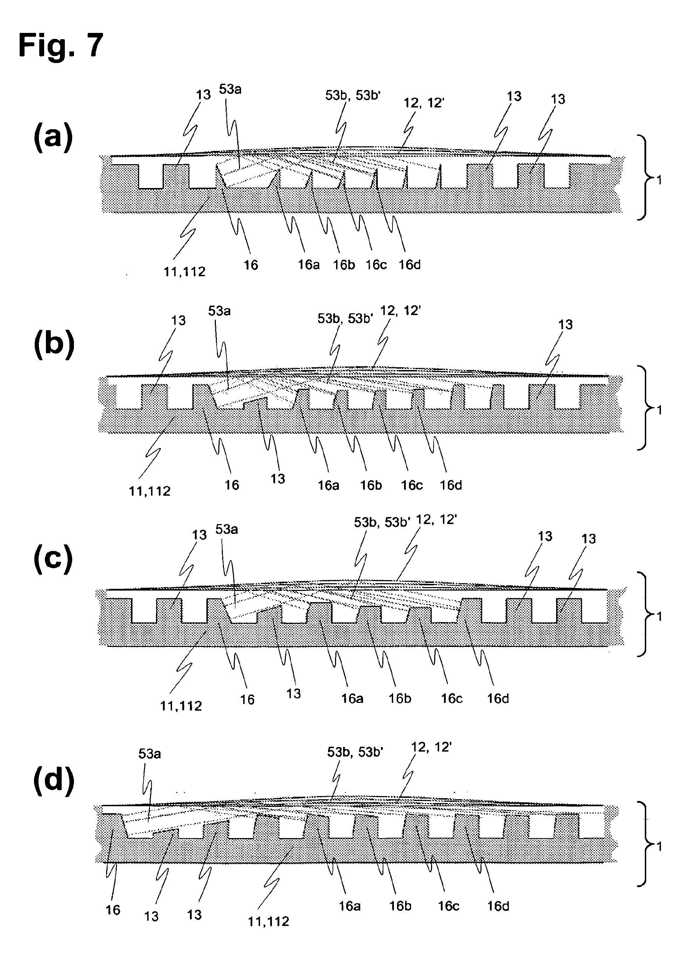

Further examples of possible prism structures are depicted in Figure 7(a) to (d). In the variants shown in Figures 7(a) and (b), the prism structures are narrow, with large prism surfaces for collecting the impinging light. Such a design is advantageous from the point of view of optical detection. However, the narrow prism-structures lead to a decrease of the flow resistance along the detection area 55, which is less advantageous for the properties of the micro-fluidic chamber 1.

In the embodiments in Figures 7(c) and (d), the prism-like structures are as broad as the other walls 13. This increases the flow resistance along the detection area. On the other hand, the surface of the prism that is available for receiving light is decreased.

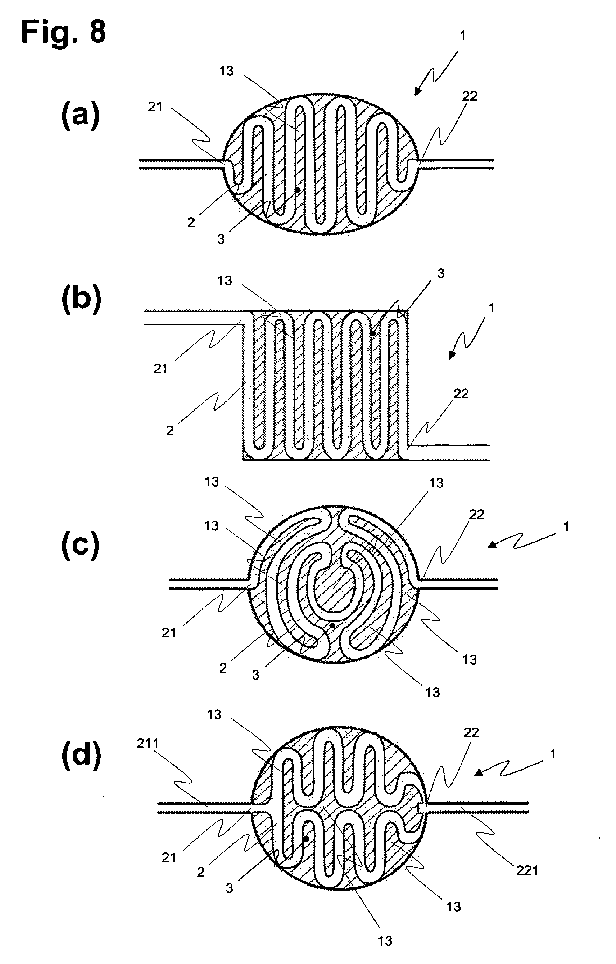

A micro-fluidic chamber may have circular shape, as in the embodiment of the sensor device shown in Figure 6, or may have any other suitable shape. The same holds true for the specific design of the fluid channel in the micro-fluidic chamber. Depending on the specific design of a sensor device according to the invention some embodiments of micro-fluidic chambers may be preferable over others. Figure 8 shows a number of possible variants of micro-fluidic chambers suitable for use with a sensor device according to the invention. In Figure 8(a) the meanders of the fluid channel 2 are arranged in an elliptically shaped chamber 1, while in the embodiment of Figure 8(b) the chamber 1 has rectangular shape. In Figure 8(c) a circularly shaped chamber 1 with an alternative course of a meandering fluid channel 12 is shown.

Instead of having only one fluid channel 2, the walls 13 of the micro-fluidic chamber may define two or more fluid channels within the chamber, extending from a common inlet to a common outlet. Figure 8(d) shows such an embodiment of a micro-fluidic chamber 1. An inlet conduit 211 opens toward the chamber 1 through a common inlet 21. The fluid channel then splits up into two separate fluid channels 2, 2', which join again at a common outlet 22. In such an embodiment preferably constructive means such as flow barriers are provided that ensure that during the filling procedure the chamber 1 is completely filled before the liquid flow proceeds further through the outlet 22.

A curved or meandering design of the fluid channel is advantageous for fluid chambers with larger base areas, since the longest possible distance between the fluid channel and an outer edge of the gap is short. In addition the meandering fluid channel can be used as an efficient means to limit the maximum flow through a fluidic system.

In the embodiments of sensor devices according to the invention discussed so far, the micro-fluidic chamber 1 comprises a bottom structure 11 and a top cover membrane 12, which are sealed together in a sealing area 15 along an outer rim of the chamber 1. Suitable materials of the bottom substrate 11 and the top cover 12 are for example polymer materials. Suitable methods for connecting the two substrates 11, 12 are thermal bonding, laser bonding, gluing etc.

The walls 13 can be realized as an integral part of the bottom substrate 11. In such a case the fluid channel 2, and even the inlet and outlet conduits can, as an example, be produced by embossing the necessary void structures into a flat bottom structure 12. To obtain the necessary gap 3 one may arrange a thin spacer layer with height H3 between bottom layer structure and top layer 12 around the chamber, or may produce the gap together with the fluid channel and the walls in the embossing step. Another suitable technology for the manufacture of micro-fluidic chambers is injection molding.

In a possible alternative approach the walls 13 are realized as separate filling structures, mounted onto a flat bottom layer 11. In that approach, a filling body may be attached to a bottom layer, and then may be arranged between said bottom layer and a adjacent top layer in a sandwich-like manner.

In an especially advantageous embodiment of a sensor device according to the invention, the wave length used for detection is chosen such that it has a high absorption coefficient in the liquid, which in most cases will be an aqueous solution. Useful wavelengths include those wavelengths where water has a high absorption coefficient, for example, 630 nm or 1400 nm. An absorption maximum for a typical insulin formulation is given at 270 nm. Thus such a wavelength is particularly advantageous for use with insulin pump systems. Although a part of the irradiated light is absorbed in the liquid medium 4, the photo sensors receive sufficient signal to deliver reliable results.

If, however, an air bubble is dragged along the fluid channel 2, and reaches the detection area 55, the absorption is strongly reduced, and the signal received by one or more of the photo sensors increases significantly. This sudden increase in signal intensity can be subsequently used to generate a warning message for the user and/or the control system of an infusion pump device that an air bubble is present in the fluidic system, and that the reliability of the sensor may be compromised. Thus, a sensor device according to the invention can also be used to detect air bubbles in a fluidic system, and to determine if its pressure measurement values may be erroneous due to the presence of an air bubble in the sensor.

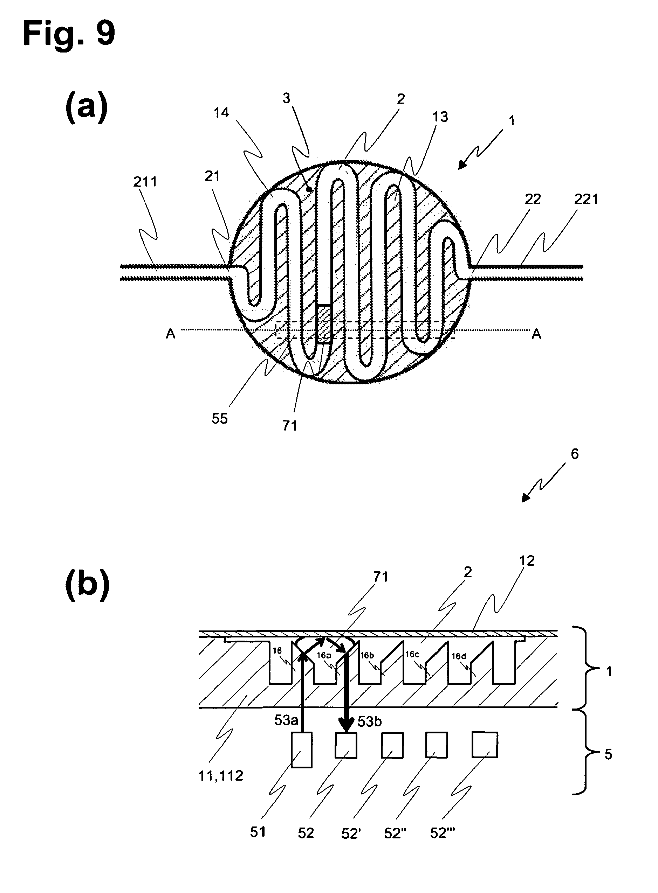

Figure 9 shows a sensor device according to the invention as shown in Figure 6 used as an air bubble sensor. An air bubble 71 in the liquid stream has entered the micro-fluidic chamber 1. For energetic reasons it cannot enter the fluid gap, and is dragged along the fluid channel 2. When the air bubble 71 reaches the detection area 55, the incident light beam 53a and the reflected light beam 53b pass through the air bubble, as shown in Fig. 9(b). The light intensity detected by the detector 52 increases, which the sensor device will interpret as the detection of an air bubble.

Taking into account the known geometry of the micro-fluidic chamber 1, the current conveying velocity of the liquid and the time period between the increase of the light intensity (corresponding to the front of the air bubble) and the decrease of the light intensity back to a normal value (marking the end of the air bubble), such a sensor device according to the invention is even able to determine the volume of the passed air bubble. Such information may then be used by a controlling unit to assess the situation and to take the necessary steps.

In an embodiment of a sensor device according to the invention particularly directed to the use as a air bubble sensor device, the membrane 12 may be realized as a semirigid membrane, or even as a rigid cover.

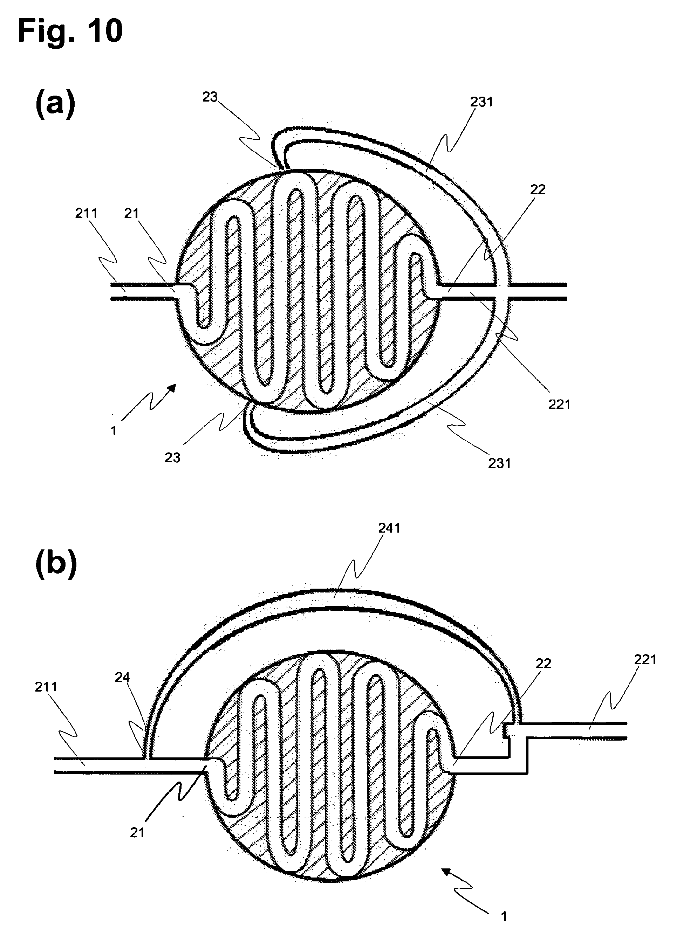

Two further advantageous variants of micro-fluidic chambers 1 for use with a sensor device 6 according to the invention are disclosed in Figure 10. In Figure 10(a) two additional outlet openings 23 are located at different positions along the meandering fluid channel 2, from which additional outlet conduits 231 branch off. Downstream of the main outlet 22, the outlet conduits 221, 231 converge again to a common outlet conduit. The additional outlet openings 23 are smaller than the main outlet opening 22. The width of the narrow outlets 23 should be 50% or less of the width of the fluid channel 2. For an air bubble with certain dimensions it will be energetically less favorable to enter the narrow outlet 23, due to the interface tensions, than to stay in the comparably wide fluid channel 2. Thus the bubbles will stay inside of the fluid channel, where they will be eventually detected by the optical detection system 5. Since three outlets 23, 22 are available, the through-put of liquid through the sensor device 6 is increased.

Figure 10(b) depicts another preferred variant of a sensor device according to the invention, with an additional conduit 241 bypassing the micro-fluidic chamber 1. Said bypass conduit 241 directly connects the inlet conduit 221 to the outlet conduit 221. The width of the inlet 24 of the bypass conduit 241 is much smaller, 50% or less, than the width of the inlet conduit 211. It is thus not favorable for an air bubble to enter the inlet 24 and bypass conduit 241, and the air bubble will enter the sensor device, where it can detected. To ensure that both the bypass conduit 241 and the micro-fluidic chamber 1 are completely filled during the first filling procedure, a flow barrier or a similar means can be used, as already discussed previously.

The present invention is not to be limited in scope by the specific embodiments described herein. Indeed, various modifications of the present invention, in addition to those described herein, will be apparent to those skilled in the art from the foregoing description and accompanying drawings. Thus, the scope of the invention is defined by the appended claims.

### List of Reference Numerals

| | |
|---|---|
| 1 | micro-fluid chamber |
| 11 | bottom substrate |
| 112 | bottom substrate material |
| 12 | flexible, resilient top cover membrane |
| 12', 12" | deformed cover membrane |
| 13 | wall, filling |
| 131 | top surface of a wall |
| 14 | inner volume |
| 15 | sealing area |
| 16, 16a, 16b, 16c, 16d | prism structure |
| 2, 2' | fluid channel |
| 21 | inlet |
| 211 | inlet conduit |
| 22 | outlet |
| 221 | outlet conduit |
| 23 | additional outlet |
| 231 | additional outlet conduit |
| 24 | inlet of the bypass conduit |
| 241 | bypass conduit |
| 3 | fluid gap |
| 3.1, 3.2, ...., 3.10 | sections of the gap |
| 4 | liquid |
| 5 | optical detection system |
| 51 | light emitting device |
| 52, 52', 52" | photo sensor |
| 53a | incident light beam |
| 53b | reflected light beam |
| 531, 531', 531" | reflection point |
| 55 | detection area |
| 6 | sensor device |
| 7 | air, gas |
| 71 | air bubble |
| H1 | height of the fluid chamber |
| H2 | height of a wall |
| H3 | height of the gap |
| Δp | pressure difference |

## Claims

1. A sensor device (6) for use in a liquid medicament delivery system, with a micro-fluidic chamber (1) comprising a rigid bottom substrate (11) and a cover (12), the substrate (11) and the cover (12) defining an inner volume (14) of the chamber, the chamber being connected to a fluidic system through which the liquid medicament can be delivered, and an optical detection system (5) that is arranged to emit one or more light beams (53a) toward the cover (12) and to observe the one or more light beams (53b) reflected from the cover (12), wherein the cover is a flexible, resilient cover membrane, and the optical detection system is arranged to determine a deformation of the cover membrane, **characterized in that** the optical detection system (5) is arranged on a side of the bottom substrate (11) opposite to the cover (12).

2. The sensor device according to claim 1, **characterized in that** the one or more light beams (53a) impinging on the cover (12) and/or the one or more reflected light beams (53b) pass through the bottom structure (11).

3. The sensor device according to claim 1 or 2, **characterized in that** the wavelength of the emitted light beams (53a) is chosen such that a liquid (4) in the micro-fluidic chamber (1) shows a high absorption coefficient at this wavelength.

4. A sensor device according to any of the preceding claims **characterized in that** the optical detection system (5) comprises two or more photo sensors (52, 52', 52") or a photo sensor array.

5. A sensor device according to any of the preceding claims **characterized in that** the sensor device is adapted such that the one or more light beams (53a) are reflected on a fluid-contacting surface of the cover (12).

6. A sensor device according to any of the preceding claims, **characterized in that** the sensor device is a pressure sensor and/or an air bubble sensor.

7. The sensor device according to any of the preceding claims, **characterized in that** the bottom structure (11) comprises prism-like structures (16, 16a, 16b, 16c) for coupling a light beam (53a, 53b) from a bottom structure material (112) into a liquid (4) in an inner volume (14) of the micro-fluidic chamber (1), and/or from the liquid (4) into the bottom structure material (112).

8. The sensor device according to any of the preceding claims, **characterized by** one or more walls or fillings (13) positioned in the chamber (1), the walls or fillings (13) defining a fluid channel (2) therebetween such that the fluid channel (2) extends from an inlet (21) of the chamber to an outlet (22) of the chamber; wherein each of the walls or fillings (13) has a height (H2) less than a height (H1) of the chamber (1) defined by the distance between the bottom substrate (11) and the cover membrane (12), so as to define a fluid gap (3) between a top surface (131) of each wall or filling (13) and the cover membrane (12), and wherein the dimensions (H1, H2) of the walls or fillings (13) and the chamber (1) are chosen such that the fluid gap (3) will be filled with liquid (4) by capillary forces via the fluid channel (2) when liquid (4) is introduced into the fluid chamber (1).

9. A sensor device according to claim 8, **characterized in that** prism-like structures (16, 16a, 16b, 16c) are provided in the walls or fillings (13) for coupling a light beam (53a, 53b) from a bottom structure material (112) of the wall (13) into a liquid (4) in an inner volume (14) of the micro-fluidic chamber (1), and/or from the liquid (4) into the bottom structure material (112).

10. A sensor device according to any of the preceding claims, **characterized in that** the fluid channel (2) has a meander-like shape.

11. A sensor device according to any of the preceding claims, **characterized by** an additional conduit (241) bypassing the chamber (1) and fluidly connecting an inlet conduit (211) and an outlet conduit (221) of the chamber (1).

12. An infusion pump device for use in a liquid medicament delivery system, **characterized by** a sensor device according to any of claims 1 to 9.

13. A liquid medicament delivery system, **characterized by** a sensor device according to any of claims 1 to 11.

14. The use of a sensor device according to any of claims 1 to 11 for measuring the pressure in a fluidic system, and/or for measuring the presence of air bubbles in a fluidic system.

## Patentansprüche

1. Sensorvorrichtung (6) zum Einsatz in einem Verabreichungssystem für flüssige Medikamente, mit einer mikrofluidischen Kammer (1), die ein starres unteres Substrat (11) und eine Abdeckung (12) umfasst, welche ein inneres Volumen (14) der Kammer abgrenzen, wobei die Kammer mit einem fluidischen System verbunden ist, durch welches das flüssige Medikament verabreicht werden kann, und mit einem optischen Detektionssystem (5), das dafür eingerichtet ist, einen oder mehrere Lichtstrahlen (53a) in Richtung der Abdeckung (12) zu emittieren, und die von der Abdeckung (12) reflektierten einen oder mehreren Lichtstrahlen (53b) zu beobachten, wobei die Abdeckung eine flexible, elastische Abdeckungsmembran ist, und das optische Detektionssystem dafür eingerichtet ist, eine Deformation der Abdeckungsmembran zu bestimmen, **dadurch gekennzeichnet, dass** das optische Detektionssystem (5) auf einer der Abdeckung (12) gegenüberliegenden Seite des unteren Substrats (11) angeordnet ist.

2. Sensorvorrichtung nach Anspruch 1, **dadurch gekennzeichnet, dass** die auf die Abdeckung auftreffenden (12) einen oder mehreren Lichtstrahlen (53a) und/oder die einen oder mehreren reflektierten Lichtstrahlen (53b) die untere Struktur (11) durchlaufen.

3. Sensorvorrichtung nach Anspruch 1 oder 2, **dadurch gekennzeichnet, dass** die Wellenlänge der emittierten Lichtstrahlen (53a) derart gewählt ist, dass eine Flüssigkeit (4) in der mikrofluidischen Kammer (1) bei dieser Wellenlänge einen hohen Absorptionskoeffizienten aufweist.

4. Sensorvorrichtung nach irgendeinem der vorstehenden Ansprüche, **dadurch gekennzeichnet, dass** das optische Detektionssystem (5) zwei oder mehr Lichtsensoren (52, 52', 52") oder ein Lichtsensorarray umfasst.

5. Sensorvorrichtung nach irgendeinem der vorstehenden Ansprüche, **dadurch gekennzeichnet, dass** die Sensorvorrichtung derart angepasst ist, dass die einen oder mehreren Lichtstrahlen (53a) auf einer mit einer Flüssigkeit in Kontakt stehenden Oberfläche der Abdeckung (12) reflektiert werden.

6. Sensorvorrichtung nach irgendeinem der vorstehenden Ansprüche, **dadurch gekennzeichnet, dass** die Sensorvorrichtung ein Drucksensor und/oder ein Luftblasensensor ist.

7. Sensorvorrichtung nach irgendeinem der vorstehenden Ansprüche, **dadurch gekennzeichnet, dass** die untere Struktur (11) prisma-artige Strukturen (16, 16a, 16b, 16c) umfasst zur Kopplung eines Lichtstrahls (53a, 53b) aus einem Material der unteren Struktur (112) in eine Flüssigkeit (4) in einem inneren Volumen (14) der mikrofluidischen Kammer (1) und/oder von der Flüssigkeit (4) in das Material der unteren Struktur (112).

8. Sensorvorrichtung nach irgendeinem der vorstehenden Ansprüche, **dadurch gekennzeichnet, dass** in der Kammer (1) eine oder mehrere Wände oder Füllungen (13) positioniert sind, wobei die Wände oder Füllungen (13) dazwischen einen Flüssigkeitskanal (2) derart definieren, dass sich der Flüssigkeitskanal (2) von einer Eintrittsöffnung (21) der Kammer zu einer Austrittsöffnung (22) der Kammer erstreckt; wobei jede der Wände oder Füllungen (13) eine Höhe (H2) aufweist, die kleiner ist als eine durch den Abstand zwischen dem unteren Substrat (11) und der Abdeckungsmembran (12) definierte Höhe (H1) der Kammer, so dass ein Flüssigkeitsspalt (3) zwischen einer oberen Oberfläche (131) jeder Wand oder Füllung (13) und der Abdeckungsmembran (12) definiert wird, und wobei die Dimensionen (H 1, H2) der Wände oder Füllungen (13) und der Kammer derart gewählt sind, dass der Flüssigkeitsspalt (3) durch Kapillarkräfte über den Flüssigkeitskanal (2) mit Flüssigkeit (4) gefüllt wird, wenn in die Flüssigkeitskammer (1) eine Flüssigkeit (4) eingeführt wird.

9. Sensorvorrichtung nach Anspruch 8, **dadurch gekennzeichnet, dass** in den Wänden oder Füllungen (13) prisma-artige Strukturen (16, 16a, 16b, 16c) vorgesehen sind zur Kopplung eines Lichtstrahls (53a, 53b) aus einem Material der unteren Struktur (112) der Wand (13) in eine Flüssigkeit (4) in einem inneren Volumen (14) der mikrofluidischen Kammer (1) und/oder aus der Flüssigkeit (4) in das Material der unteren Struktur (112).

10. Sensorvorrichtung nach irgendeinem der vorstehenden Ansprüche, **dadurch gekennzeichnet, dass** der Flüssigkeitskanal (2) eine mäanderförmige Gestalt aufweist.

11. Sensorvorrichtung nach irgendeinem der vorstehenden Ansprüche, **gekennzeichnet durch** eine zusätzliche Leitung (241), welche die Kammer (1) überbrückt und eine Fliessverbindung zwischen einer Eintrittsleitung (211) und einer Austrittsleitung (221) der Kammer (1) bildet.

12. Infusionspumpenvorrichtung zum Einsatz in einem Verabreichungssystem für flüssige Medikamente, **gekennzeichnet durch** eine Sensorvorrichtung nach einem der Ansprüche 1 bis 9.

13. Verabreichungssystem für flüssige Medikamente, **gekennzeichnet durch** eine Sensorvorrichtung nach einem der Ansprüche 1 bis 11.

14. Verwendung einer Sensorvorrichtung nach irgendeinem der Ansprüche 1 bis 11 zur Messung des Drucks in einem fluidischen System und/oder zur Messung des Vorkommens von Luftblasen in einem fluidischen System.

## Revendications

1. Dispositif de capteur (6) destiné à être utilisé dans un système d'administration de médicament liquide, ayant une chambre microfluidique (1) comprenant un substrat inférieur rigide (11) et un couvercle (12), le substrat (11) et le couvercle (12) définissant un volume intérieur (14) de la chambre, la chambre étant reliée à un système fluidique à travers lequel le médicament liquide peut être administré, et un système de détection optique (5) qui est agencé pour émettre un ou plusieurs faisceaux lumineux (53a) vers le couvercle (12) et pour observer le ou les faisceaux lumineux (53b) réfléchis par le couvercle (12), la couverture étant une membrane de couvercle élastique, flexible, et le système de détection optique étant agencé pour déterminer une déformation de la membrane de couvercle, **caractérisé par le fait que** le système de détection optique (5) est agencé sur un côté du substrat inférieur (11) opposé au couvercle (12).

2. Dispositif de capteur selon la revendication 1, **caractérisé par le fait que** le ou les faisceaux lumineux (53a) frappant le couvercle (12) et/ou le ou les faisceaux lumineux réfléchis (53b) passent à travers la structure inférieure (11).

3. Dispositif de capteur selon la revendication 1 ou 2, **caractérisé par le fait que** la longueur d'onde des faisceaux lumineux émis (53a) est choisie de telle sorte qu'un liquide (4) dans la chambre microfluidique (1) présente un coefficient d'absorption élevé à cette longueur d'onde.

4. Dispositif de capteur selon l'une quelconque des revendications précédentes, **caractérisé par le fait que** le système de détection optique (5) comprend deux photodétecteurs ou plus (52, 52', 52") ou une rangée de photodétecteurs.

5. Dispositif de capteur selon l'une quelconque des revendications précédentes, **caractérisé par le fait que** le dispositif de capteur est agencé de telle sorte que le ou les faisceaux lumineux (53a) sont réfléchis sur une surface du couvercle (12) en contact avec du fluide.

6. Dispositif de capteur selon l'une quelconque des revendications précédentes, **caractérisé par le fait que** le dispositif de capteur est un capteur de pression et/ou un capteur de bulles d'air.

7. Dispositif de capteur selon l'une quelconque des revendications précédentes, **caractérisé par le fait que** la structure inférieure (11) comprend des structures de type prisme (16, 16a, 16b, 16c) pour coupler un faisceau lumineux (53a, 53b) provenant d'une matière de structure inférieure (112) dans un liquide (4) dans un volume intérieur (14) de la chambre microfluidique (1), et/ou provenant du liquide (4) dans la matière de structure inférieure (1 1 2).

8. Dispositif de capteur selon l'une quelconque des revendications précédentes, **caractérisé par** une ou plusieurs parois ou bourrages (13) positionnées dans la chambre (1), les parois ou bourrages (13) définissant un canal fluidique (2) entre celles-ci de telle sorte que le canal fluidique (2) s'étend d'une entrée (21) de la chambre à une sortie (22) de la chambre; chacune des parois ou bourrages (13) ayant une hauteur (H2) inférieure à une hauteur (H1) de la chambre (1) définie par la distance entre le substrat inférieur (1) et la membrane de couvercle (12), de manière à définir une fente fluidique (3) entre une surface supérieure (131) de chaque paroi ou bourrage (13) et la membrane de couvercle (12), et les dimensions (H 1, H2) des parois ou bourrages (13) et de la chambre (1) étant choisies de telle sorte que la fente fluidique (3) sera rempli de liquide (4) par des forces capillaires par l'intermédiaire du canal fluidique (2) lorsque du liquide (4) est introduit dans la chambre fluidique (1).

9. Dispositif de capteur selon la revendication 8, **caractérisé par le fait que** des structures de type prisme (16, 16a, 16b, 1 6c) sont disposées dans les parois ou bourrages (13) pour coupler un faisceau lumineux (53a, 53b) provenant d'une matière de structure inférieure (112) de la paroi (13) dans un liquide (4) dans un volume intérieur (14) de la chambre microfluidique (1), et/ou provenant du liquide (4) dans la matière de structure inférieure (112).

10. Dispositif de capteur selon l'une quelconque des revendications précédentes, **caractérisé par le fait que** le canal fluidique (2) a une forme de type méandres.

11. Dispositif de capteur selon l'une quelconque des revendications précédentes, **caractérisé par** une conduite supplémentaire (241) contournant la chambre (1) et reliant de manière fluidique une conduite d'entrée (211) et une conduite de sortie (221) de la chambre (1).

12. Dispositif de pompe à perfusion destiné à être utilisé dans un système d'administration de médicament liquide, **caractérisé par** un dispositif de capteur selon l'une quelconque des revendications 1 à 9.

13. Système d'administration de médicament liquide, **caractérisé par** un dispositif de capteur selon l'une quelconque des revendications 1 à 11.

14. Utilisation d'un dispositif de capteur selon l'une quelconque des revendications 1 à 11 pour mesurer la pression dans un système fluidique et/ou pour mesurer la présence de bulles d'air dans un système fluidique.
